# EUROPÄISCHE PATENTSCHRIFT

(11) **EP 1 351 772 B1**
(45) Veröffentlichungstag und Bekanntmachung des Hinweises auf die Patenterteilung: **07.06.2006**
(21) Anmeldenummer: 01272637.8
(22) Anmeldetag: 08.12.2001
(51) Int. Cl.: B04B 5/04

(54) **ZENTRIFUGE MIT BLUTBEUTELSYSTEM MIT OBEREM UND UNTEREM ABGANG**
CENTRIFUGE COMPRISING A BLOOD BAG SYSTEM WITH AN UPPER AND LOWER OUTLET
CENTRIFUGEUSE COMPORTANT UN SYSTEME DE POCHE DE SANG PRESENTANT UNE SORTIE SUPERIEURE ET UNE SORTIE INFERIEURE

(30) Priorität: 29.12.2000 DE 10065283
(43) Veröffentlichungstag der Anmeldung: 15.10.2003
(73) Patentinhaber: Andreas Hettich GmbH & Co. KG, 78532 Tuttlingen (DE)
(72) Erfinder: EBERLE, Günter, 78532 Tuttlingen (DE)
(74) Vertreter: Riebling, Peter
(86) Internationale Anmeldenummer: PCT/EP2001/014440
(87) Internationale Veröffentlichungsnummer: WO 2002/053292

(56) Entgegenhaltungen:
- US-A- 4 322 298
- US-A- 4 482 342
- US-A- 4 608 178

## Beschreibung

Die Erfindung betrifft eine Zentrifuge mit Blutbeutelsystem mit oberem und unterem Abgang zur Trennung von Blutkomponenten nach dem Oberbegriff des unabhängigen Patentanspruches 1. Derartige Zentrifugen sind unter anderen bekannt aus US-A-4 482 342 und US-A-4 322 298.

Mit der auf den gleichen Anmelder zurückgehenden EP 0 026 417 B1 ist eine Zentrifuge mit Blutbeutelsystem bekannt geworden, bei dem der Blutbeutel lediglich einen oberen Abgang aufweist, was mit dem Nachteil verbunden ist, daß die Gewinnung des Plasmas schwierig ist.

Außerdem handelt es sich um ein ausschwingendes Gehänge, bei dem sich die Phasengrenzen zwischen den einzelnen Komponenten in einer senkrechten Ebene während des Zentrifugiervorganges bilden.

Demgegenüber beschreibt die EP 0 350 495 B1 ein stehendes Blutbeutelsystem. Bei diesem stehenden System besteht aber wiederum der Nachteil, daß die Trennung der Komponenten schwierig ist, weil eine Anzapfung eines, eine obere und untere Anschlußleitung aufweisenden, Blutbeutels von der Mitte her erfolgt. Dies erfordert den Einsatz eines speziell und damit teuer hergestellten Blutbeutels, der im übrigen noch anfällig gegen ein Aufplatzen ist.

Es werden also nicht die oberen und unteren Anschlüsse des Blutbeutels für die Ableitung der während des Zentrifugiervorganges gewonnenen Komponenten verwendet, sondern ein eigener, mittlerer und speziell hergestellter Abgang.

Der Erfindung liegt deshalb die Aufgabe zugrunde, eine Zentrifuge mit stehendem Blutbeutelsystem gemäß der EP 0 350 495 B1 so weiterzubilden, daß handelsübliche Blutbeutel verwendet werden können, ohne daß die Notwendigkeit besteht, Spezialanfertigungen der Beutel zu verwenden.

Der Erfindung liegt die weitere Aufgabe zugrunde, die Gewinnung der einzelnen Komponenten mit wesentlich höherem Wirkungsgrad, funktionsgerecht und mit hohem Reinheitsgrad zu erreichen.

Zur Lösung der gestellten Aufgabe ist die Erfindung durch die technische Lehre des unabhängigen Anspruches 1 gekennzeichnet.

Wesentliches Merkmal der Erfindung ist, daß ein Vollblutbeutel mit oberen und unteren Abgängen verwendet wird, der in stehender Ausführung im Rotor angeordnet ist und daß der eine Abgang, beispielsweise der obere Abgang dieses Vollblutbeutels, in einer radialen Richtung geknickt ist und der gegenüberliegende Abgang des Blutbeutels in der anderen radialen Richtung geknickt ist, so daß insgesamt der Blutbeutel in seiner Funktionslage mit seinen Abgängen eine etwa Z- oder S-förmige Form einnimmt.

Mit der gegebenen technischen Lehre ergibt sich der wesentliche Vorteil, daß nun mit einem handelsüblichen Blutbeutel mit oberen und unteren Abgängen gearbeitet werden kann, ohne daß es der Notwendigkeit bedarf, einen Spezialanschluß an diesem Blutbeutel zu verwenden.

Es hat sich nämlich gezeigt, daß durch diese Z- bzw. S-förmige Anordnung des stehenden Blutbeutels im Rotor der Zentrifuge es nun erstmals möglich ist, in sauberer Weise beispielsweise den oberen Abgang, der radial einwärts gerichtet ist, zur Gewinnung des sich in dieser radial einwärts liegenden Ebene bildenden Plasmas zu verwenden, während dem gegenüberliegend, radial auswärts der Abgang zur Gewinnung der sich ebenfalls in dieser Phasengrenze befindlichen Erythrozyten verwendet wird.

Damit besteht der weitere Vorteil, daß das eigentlich unerwünschte sogenannte "Buffycoat" in dem Vollblutbeutel verbleiben kann und sich nicht mit den anderen Komponenten vermischt.

Es bestehen nämlich eine Reihe von anderen bekannten Erfindungen nach dem Stand der Technik, bei denen das Buffycoat in andere Beutel übergeleitet werden muß, um eine saubere Trennung zwischen den Erythrozyten und dem Plasma zu erreichen. Dies vermeidet die Erfindung.

Die Erfindung sieht vor, daß das unerwünschte Buffycoat im Vollblutbeutel selbst verbleibt und daß der Blutbeutel handelsüblich ist, das heißt, er hat obere und untere Anschlußleitungen, die nun erfindungsgemäß durch die spezielle Formgebung des Blutbeutels in der Zentrifuge für die Ableitung der einzelnen sich während des Zentrifugiervorganges bildenden Komponenten verwendet werden.

Es wird allgemein darauf hingewiesen, daß die in Z- oder S-Form geformten Blutbeutel während des Zentrifugiervorganges in dem Rotor in jeder beliebigen Halterung gelagert werden kann. Es kann also jede beliebige Halterung verwendet werden, die in der Lage ist, den Blutbeutel in einer Z- oder S-Form zu halten, bei dem beispielsweise die obere Anschlußleitung radial nach innen und die untere Anschlußleitung radial nach außen verformt wird.

Nach der Erfindung wird aber ein Mehrkammersystem bevorzugt, bei dem der so geformte Blutbeutel in einem Einsatz gehalten wird, welcher Einsatz seinerseits in einer Kassette gehalten ist.

Durch dieses Einsatz-Kassetten-System ergeben sich mehrere Vorteile:

Ein erster Vorteil ist, daß ein relativ einfach aufgebauter Einsatz verwendet werden kann, in dessen einer Kammer der Vollblutbeutel in der beschriebenen Z- oder S-Form eingehängt ist und die Anschlüsse in der Z- oder S-Konfiguration abgeleitet werden, wobei in diesem Einsatz noch weitere Kammern für die Einhängung eines, zur Gewinnung der Erythrozyten dienenden, Beutels und eine weitere Kammer für einen Beutel zu Gewinnung des Plasma vorgesehen ist.

Ferner reicht es aus, diesen Einsatz nur noch mit entsprechend gesteuerten Klemmen zu versehen, wobei diese Klemmen in einem bestimmten Zentrifugierstadium öffnen und die Überleitung der im Blutbeutel hergestellten Komponenten in die einzelnen Satelliten-Beutel ermöglichen.

Diese Klemmen sind bevorzugt handgesteuert, wenn es darum geht, daß im Stillstand das ganze System in den Einsatz eingelegt wird, weil diese Klemmen dann über eine Handbetätigung an den Schläuchen eine Klemmverbindung herstellen, um die entsprechenden Zuführungs- und Abführungsschläuche abzuklemmen.

Während des Zentrifugiervorganges sind also diese Klemmen federbelastet geschlossen und werden lediglich in einem bestimmten Verfahrensstadium durch eine fremdgesteuerte Kraft geöffnet. Diese Kraft wirkt nur für einen bestimmten Zeitraum, der sensorgesteuert erfaßt wird, um zu gewährleisten, daß die gebildeten Komponenten vollständig und praktisch rückstandsfrei in die Satelliten-Beutel übergeleitet werden.

Hierbei kann als ferngesteuerte Kraft für die Ansteuerung der Klemmen ein Hubzylinder verwendet werden, ein Elektromagnet, ein Drehschieber, oder sonstige Betätigungselemente, die ferngesteuert bedienbar sind.

Hierbei ist also wichtig, daß der Einsatz für die mit der Ausbildung der drei Kammern und mit der Verwendung der beschriebenen Klemmen möglichst einfach und kostengünstig ausgebildet ist.

Die eigentlichen Ansteuerungselemente für die Ansteuerung der Klemmen sind hierbei in einer Kassette angeordnet, wobei diese Kassette etwa sektorförmig ausgebildet ist und eine Vielzahl von Kassetten gleichmäßig am Umfang verteilt am Rotor angeordnet sind.

Diese Kassetten sind über die Rotornabe mit Energieanschlüssen versehen. Jede Kassette weist hierbei eine Steckbuchse auf, und an der Rotornabe ist ein entsprechender Gegenstecker angeordnet, wobei über diese Steckverbindung zwischen Rotornabe und Kassette nun die erforderlichen Signalleitungen und die Luftleitungen eingespeist werden.

Selbstverständlich ist es möglich, statt einer zusammenhängenden Steckverbindung für die Einleitung von Energie und Luft auch zwei getrennte Steckverbindungen pro Kassette vorzusehen, wobei bei der einen Steckverbindung die Luft und bei der anderen Steckverbindung die elektrische Energie zugeführt wird.

Es werden lediglich Signalleitungen für einen Sensor zugeführt, welcher Sensor die Überwachung der Überleitung des Plasmas in den Satellitenbeutel überwacht und der die zugeordnete Klemme ansteuert, wenn er eine Färbung mit einer andersartigen Komponente in der Überleitungsleitung entdeckt.

In der Ableitung für die Gewinnung der Erythrozyten ist eine gleiche Klemme angeordnet, die ebenfalls im Ruhezustand handbetätigbar ist und die während des Zentrifugiervorganges ebenfalls durch ein fernbedienbares Bestätigungselement angesteuert wird.

Wichtig hierbei ist also, daß die Kassetten, welche die Bestätigungselemente für die darin ruhenden Einsätze beinhalten, relativ fest mit dem Rotor verschraubt sind, und sozusagen den Maschinen- und Betätigungsteil der Einsätze bilden, während die Einsätze möglichst einfach ausgebildet sein sollen.

Bei Beschädigung eines derartigen Einsatzes ist dieser also sehr leicht auszutauschen, ebenso dann, wenn es beispielsweise zum unbeabsichtigten Defekt eines oder mehrerer Beutel während des Zentrifugiervorganges kommt.

Im übrigen ist vorgesehen, daß die Kassetten, welche die Einsätze aufnehmen, mit einem durchsichtigen Deckel verschließbar sind, wobei dieser Deckel verriegelbar ist.

Damit besteht nämlich der Vorteil, daß während des Zentrifugiervorganges von oben her - durch einen ebenfalls durchsichtigen Rotordeckel - die Überleitung des Plasmas von dem Vollblutbeutel in den benachbarten Plasmabeutel mittels eines Stroboskops sehr gut beobachtet werden kann, um so die Funktion des Sensors, der die Überleitung des Plasmas überwacht, zu kontrollieren.

Dieses Stroboskop ist in Abhängigkeit von der Drehzahl des Rotors drehzahlgesteuert .

Zusätzlich zu den hier beschriebenen, ferngesteuert betätigbaren Klemmen ist es weiterhin vorgesehen, daß auch eine Schweißvorrichtung eingebaut ist, die dafür sorgt, daß nach der vollständigen Befüllung des Plasmabeutels und des Erythrozytenbeutels die zugehörenden Rohrleitungen oder Zuführschläuche abgetrennt und zugeschweißt werden.

Auf diese Weise wird also eine vollautomatische Gewinnung von Plasma und Erythrozyten erreicht.

Mit der gegebenen technischen Lehre ergibt sich also der Vorteil, daß Dank einer stehenden Anordnung des Vollblutbeutels und dank der Möglichkeit der Verwendung eines üblichen Vollblutbeutels mit oberen und unteren Anschlußleitungen nun erstmals die Möglichkeit besteht, ein besonders reines Plasma zu gewinnen, weil nur durch eine Z-förmige Verformung des Vollblutbeutels in der Zentrifugenkammer der Zentrifuge dafür gesorgt wird, daß der eine Abgang des Vollblutbeutels radial einwärts gerichtet ist und damit die sich radial einwärts ergebende Komponente im Blutbeutel zur Ableitung bringt, während andererseits der radial auswärts gerichtete Anschluß am Blutbeutel nun derart Z-förmig nach außen verformt wird, daß er nur und allein für die Ableitung der sich in radialer Richtung auswärts bildenden Komponente bestimmt und geeignet ist.

Bei der vorliegenden Erfindung erscheint noch wesentlich, daß radial innenliegend ein Druckmodul angeordnet ist, welches beispielsweise mit einem mit preßluftaufblasbarem Kissen arbeitet.

Dieses Druckluftmodul arbeitet auf einen Preßschieber, der in einer radialen Richtung von innen nach außen hin gegen den Blutbeutel bewegbar ist.

Daher wird der Blutbeutel radial einwärts von dem Preßschieber beaufschlagt und legt sich radial auswärts an einer zugeordneten Trennwand in dem Einsatz an.
Der so unter Druck gesetzte Blutbeutel verdrängt das Plasma über den Zuführungsschlauch und das geöffnete Ventil (Klemme) in den benachbarten Satellitenbeutel, in dem das Plasma aufgefangen wird.

Dementsprechend wird nach dem Ableiten der Plasmakomponente das obere Ventil wieder geschlossen, und es wird danach das untere Ventil für die Ableitung der Erythrozyten geöffnet und unter weiterer Einwirkung dieses Preßschildes wird auch die Erythrozytenkomponente bei geöffneter Klemme in den zugeordneten Erythrozytenbeutel abgeleitet.

Versuche haben ergeben, daß bei der Ableitung der Erythrozytenkomponenten nicht unbedingt die Einwirkung des Preßschildes notwendig ist; es kann ausreichen, daß allein die Zentrifugalkraft einwirkt, um eine Ableitung der Erythrozytenkomponente aus dem Vollblutbeutel in den Erythrozytenbeutel zu ermöglichen.

Im folgenden wird die Erfindung anhand von Zeichnungen näher erläutert, welche lediglich einen Ausführungsweg darstellen.. Hierbei gehen aus den Zeichnungen und ihrer Beschreibung weitere Merkmale und Vorteile der Erfindung hervor.

Es zeigen:
- Figur 1:: perspektivische Draufsicht auf einen Rotor mit einer einzigen, dort befestigten Kassette;
- Figur 2:: die gleiche Darstellung wie Figur 1 mit einer Vielzahl von befestigten Kassetten, wobei der Übersichtlichkeit halber ein Kassettenplatz freigelassen ist;
- Figur 3:: eine Kassette nach den Figuren 1 und 2 in perspektivischer Rückansicht;
- Figur 4:: die Kassette nach Figur 3 mit Darstellung weiterer Einzelheiten;
- Figur 5:: ein Einsatz zur Verwendung in der Kassette von der Bodenseite her gesehen;
- Figur 6.: der Einsatz nach Figur 5 von der Deckelseite her gesehen;
- Figur 7:: der Einsatz nach Figur 6 von der Außenseite und oben gesehen;
- Figur 8:: der Einsatz nach Figur 7 direkt von oben gesehen;
- Figur 9:: schematisiert einen Schnitt durch eine Kassette mit einem dort eingebauten Einsatz;
- Figur 10:: Schnitt durch eine Anordnung nach Figur 9 mit Darstellung weiterer Einzelheiten;
- Figur 11:: schematisiert die Stellung des Vollblutbeutels während des Zentrifugiervorganges;
- Figur 12:: eine gegenüber Figur 11 abgewandelte Ausführungsform.

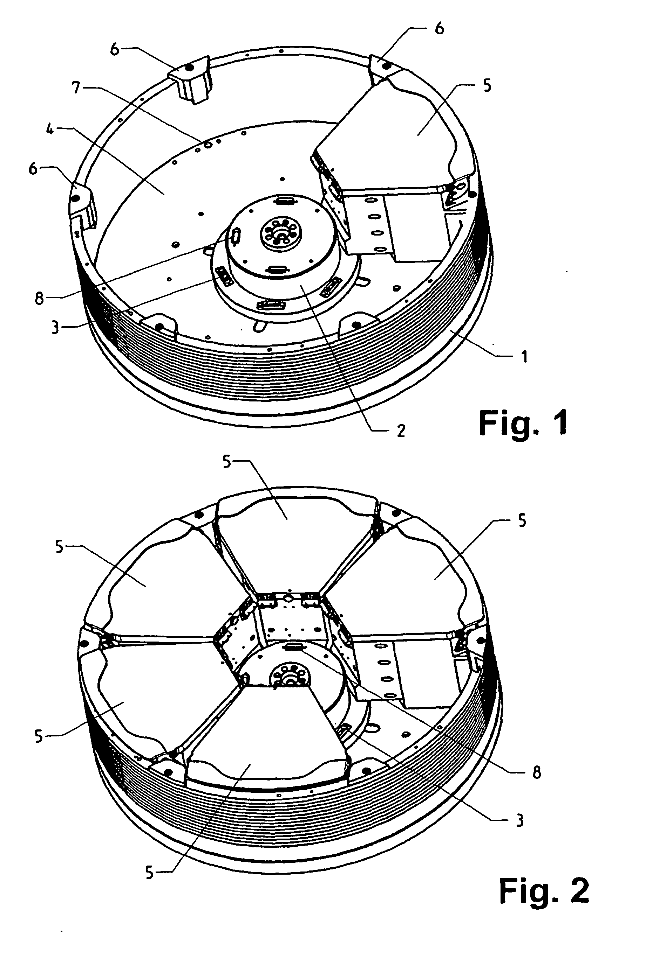

Gemäß Figur 1 ist in einem Rotor 1 eine Nabe 2 angeordnet, welche mehrere Steckanschlüsse 3, 8 zeigt.

Über die einen Steckanschlüsse 3 kann beispielsweise die Energie für die jeweilige dort gehaltene Kassette 5 zugeführt werden, während über die anderen Steckanschlüsse 8 die Luftversorgung stattfindet.

Es kann jedoch auch vorgesehen sein, daß beispielsweise die Steckanschlüsse 8 entfallen und die Luft und die elektrische Energie lediglich über die Steckanschlüsse 3 zugeführt wird.

Es ist eine Vielzahl von Kassetten 5 gleichmäßig im Innenraum 4 des Rotors angeordnet, wobei diese Kassetten lösbar zwischen, einzelnen am Umfang des Rotors gehaltenen, Arretierstiften 6 eingebaut sein können.

In einer anderen Ausführungsform können diese Kassetten 5 jedoch auch fest mit dem Rotor verbunden sein. Hierzu sind im Rotorboden entsprechende Bohrungen 7 vorhanden, durch welche Schrauben hindurchgreifen, die mit dem Boden der jeweiligen Kassetten 5 verbunden sind.

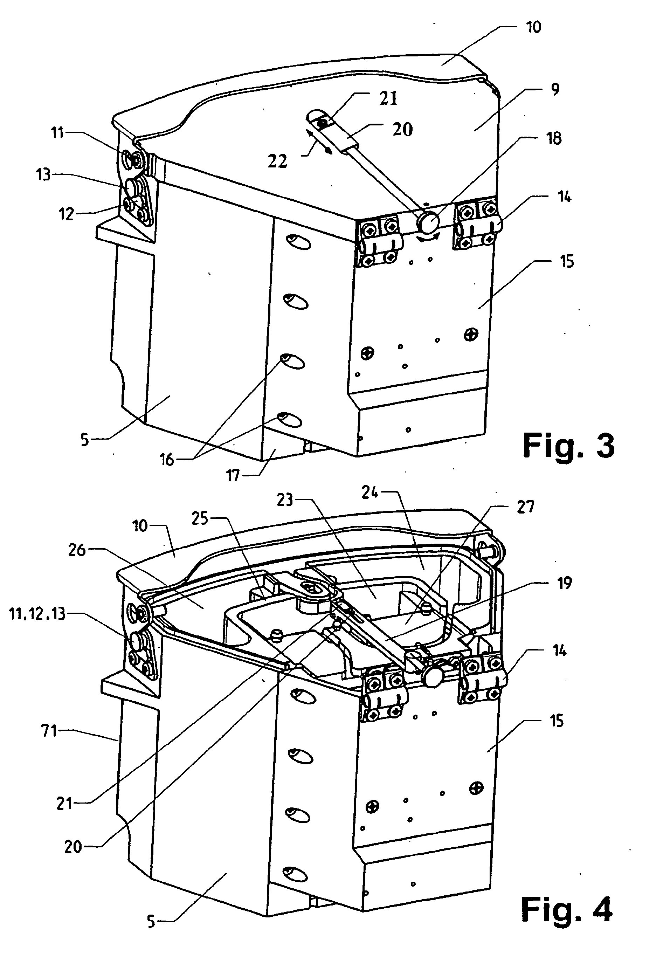

Die Figuren 3 und 4 zeigen eine derartige Kassette 5 mit weiteren Einzelheiten.

Sie besteht im wesentlichen aus einer Außenwand 71 die kraft- und formschlüssig an der Innenwand des Rotors 1 anliegt sowie einem der Außenwand 71 gegenüberliegenden Rückteil 15, welches radial einwärts zur Nabe gerichtet ist.

An der Unterseite des Rückteils 15 ist der beschriebene Gegenstecker angeordnet, der mit den Steckanschlüssen 3, 8 zusammenwirkt.

Die Kassette 5 kann daher von oben her in den Rotor eingesetzt werden und der am Bodenteil des Rückteils 15 angeordnete Gegenstecker rastet dann mit den Steckanschlüssen 3, 8 zusammen.

Das Rückteil 15 ist mittels Schrauben 16 mit der Rückwand 17 der Kassette 5 verbunden.

Der Deckel 9 der Kassette 5 ist durchsichtig ausgebildet und trägt an seiner Vorderseite (radial auswärts gerichtet) eine Verschlußklappe 10, die einen in einer Schwenkachse 11 gehaltenen Riegel 12 trägt, der mit zugeordneten Bolzen 13 verriegelbar ist.

Das Schwenkscharnier 14 des Deckels 9 ist am Rückteil 15 angeordnet.

Die Figur 4 zeigt im übrigen, daß in die Kassette 5 ein Einsatz 25 eingesetzt ist, wobei die Führung zwischen der Kassette 5 und dem Einsatz 25 so vorgesehen ist, daß er lose und mit Spiel in die Kassette eingesetzt werden kann.

Nachdem - wie beschrieben - im Rückteil 15 der Kassette die Betätigungselemente für den Einsatz angeordnet sind, kommt es dabei zu einer Gegenüberstellung der Betätigungselemente zu den zugeordneten Flächen am Einsatz 25. Hierauf wird noch näher eingegangen.

Anhand der Figur 3 kann noch erläutert werden, daß die Kassette im Deckel 9 eine Führungshülse 20 trägt, in der in den Pfeilrichtungen 22 verschiebbar ein Sensor 21 angeordnet ist.

Die Verschiebung des Sensors 21 erfolgt hierbei über ein Welle 19, die als Spindel ausgebildet ist und die drehfest mit einem Einstellrad 18 verbunden ist.

Auf diese Weise kann also die Stellung des Sensors 21 in radialer Richtung stufenlos im Deckel 9 eingestellt werden. Gemäß Figur 4 zeigt der Einsatz 25 drei voneinander getrennte und durch Wandungen voneinander abgeteilte Kammern 23, 24, 26.

Die Kammer 23 dient zur Aufnahme des Vollblutbeutels 31, während die Kammer 24 zur Aufnahme des Plasmabeutels 45 dient, während die Kammer 26 zur Aufnahme des Erythrozytenbeutels 44 dient.

Die dort dargestellte Adapterplatte 27 zeigt Schlauchführungskanäle, die später noch beschrieben werden und im übrigen Halterungen für das Einhängen des Vollblutbeutels 31 in Form von Stiften. Diese Adapterplatte 27 ist bevorzugt lösbar im Einsatz 25 gehalten.

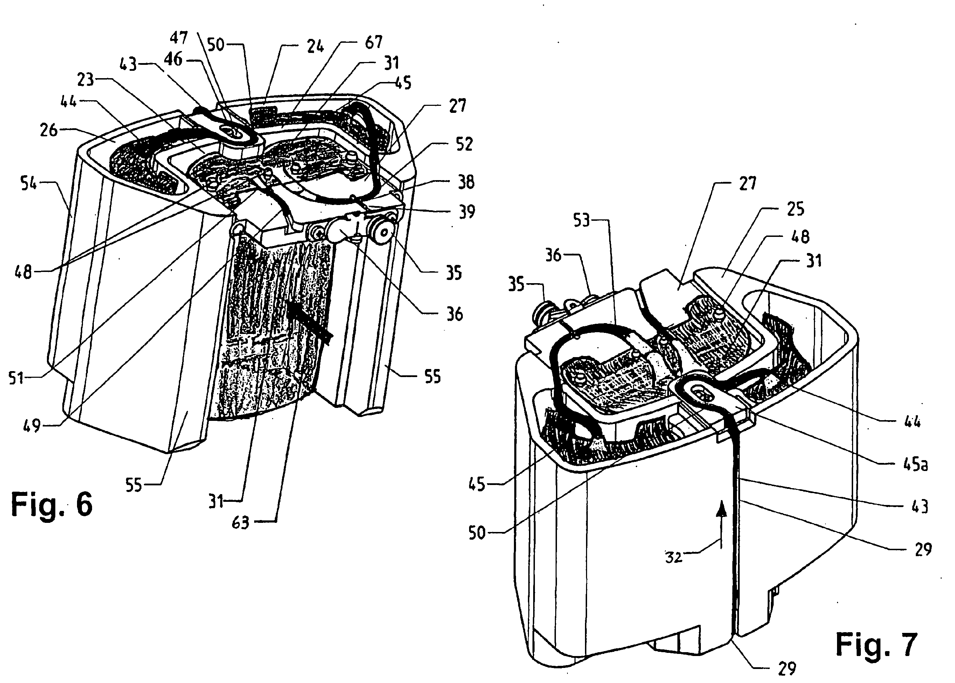

Figur 5 zeigt nun einen derartigen Einsatz 25 von der Bodenseite her. Hierbei ist erkennbar, daß der Vollblutbeutel 31 oben eingehängt ist und zwar an der Adapterplatte 27, während sein unterer Ablauf in Form eines Schlauches 43 zwischen einer Klemmvorrichtung abgeleitet wird. Der Vollblutbeutel 31 ist im übrigen mit seinen unteren Anschlußlappen ebenfalls an Stiften 42 eingehängt, die im Bodenbereich des Einsatzes 25 angeordnet sind.

Die übrigen Kammern 24, 26 des Einsatzes 25 sind durch entsprechende Böden 33, 34 abdichtend nach unten abgeschlossen.

Die an der Bodenseite angeordnete Klemmvorrichtung besteht im wesentlichen aus einer Handbetätigung 30 (Handrad), welche über eine Welle oder Achse mit einem in einer Langlochführung 41 verschiebbaren Klemme 40 verbunden ist, wobei das Handrad 30 mit einer nicht näher dargestellten Schließfeder in Schließstellung vorbelastet wird, so daß die Klemme 40 ständig den Schlauch 43 im Schlauchführungskanal 29 geschlossen hält.

Lediglich wenn ein im Rückteil 15 der Kassette 5 angeordneter Stößel als Hubzylinder auf diese Handbetätigung 30 wirkt, wird die Klemme 40 in Öffnungsstellung verschoben und damit der Durchfluß im Schlauch 43 im Schlauchführungskanal 29 freigegeben.

Die gesamte Anordnung ist also in der Bodenseite 28 dieses Einsatzes 25 angeordnet, im übrigen ist noch erkennbar, daß an der gegenüberliegenden Seite des Vollblutbeutels 31 auch die Adapterplatte 27 mit einer dementsprechend von Hand betätigbaren Klemme versehen ist, die auch hier aus einer Handbetätigung 35 besteht, die federbelastet in der Schließstellung gehalten wird, wobei diese Handbetätigung 35 am freien verschwenkbaren Ende einer Wippe 36 gehalten ist, die in einer Schwenkachse 37 verschwenkbar ist.

Die Figuren 6 und 7 zeigen weitere Einzelheiten im Kopfbereich des Einsatzes 25. Dort ist gemäß Figur 7 erkennbar, daß der aus dem Bodenbereich des Vollblutbeutels 31 herausgeführte Schlauch 43 über einen Schlauchführungskanal 29 in Pfeilrichtung 32 nach oben geführt ist und in einen Schlauchkanal 50 eintritt, der im Bereich einer Schlauchführung 45a angeordnet ist.

Diese Schlauchführung 45a ist in radialer Richtung einstellbar und feststellbar ausgebildet. Hierbei greift eine Führungsschraube 47 durch ein Langloch 46 hindurch und ist in diesem Langloch feststellbar.

Mit der radialen Einstellung der Schlauchführung wird der Überlauf in radialer Richtung für den Schlauch 43 eingestellt. Hiermit wird bestimmt, wieviel Rest-Erythrozyten im Buffycoat 57 noch verbleiben sollen.

Der Schlauch 43 stellt also die Verbindung zwischen dem Vollblutbeutel 31 und dem für die Gewinnung der Erythrozyten bestimmten Erythrozytenbeutel 44 her.

Im übrigen ist in Figur 6 erkennbar, wie der zur Gewinnung des Plasmas dienende Schlauch 53 aus dem Kopfbereich des Vollblutbeutels 31 heraustritt, dort in radialer Richtung einwärtsgebogen ist und über die Adapterplatte 27 unter Zwischenschaltung der Klemme 35, 36, 38 in den zur Gewinnung des Plasmas dienenden Plasmabeutel 45 in der Kammer 24 führt.

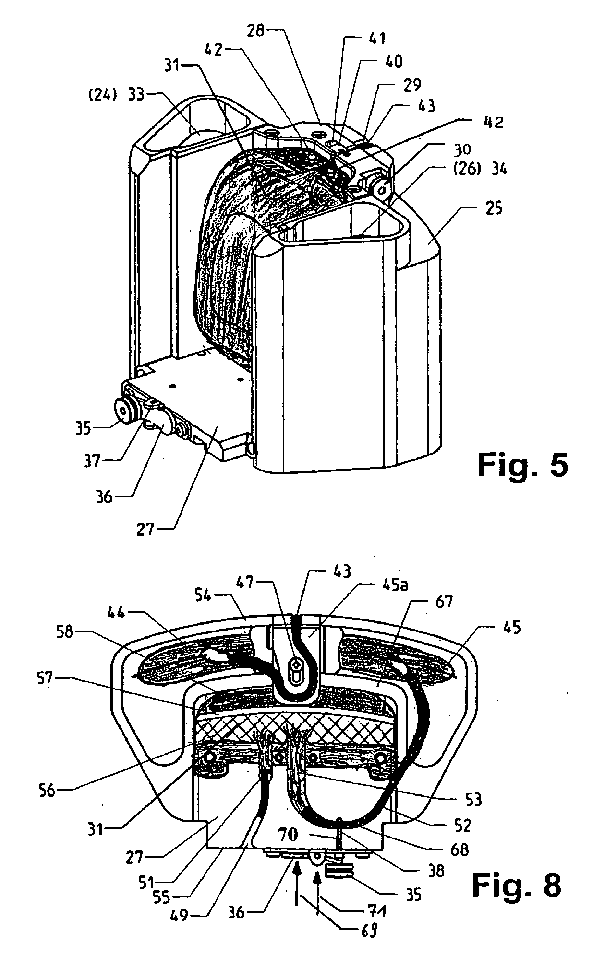

Die Klemme für den fernbetätigbaren und den handbetätigbaren Abschluß des Schlauches 53 zur Gewinnung des Plasmas ist in Figur 8 gut erkennbar und besteht im wesentlichen aus einer, mittels einer Feder 70 in Schließstellung vorgespannten, Klemme 38, die an einen ortsfesten Stift (Anschlagstift 68) sich anlegt. Damit wird federbelastet die Klemme stets in Schließstellung gehalten und der Plasmaschlauch 53 wird ständig abgeschlossen. Die öffnungsbewegung kann hierbei handbetätigt oder fernbetätigt erfolgen.

Für die Handbetätigung ist hierbei eine Wippe 36 vorgesehen, die in einer Schwenkachse schwenkbar gelagert ist und die bei einer Einwirkung einer Betätigungskraft 69 die Handbetätigung 35 entgegen der Kraft der Feder 70 abhebt und die Klemme 38 damit öffnet.

Anstatt der Handbetätigung, die nur im Stillstand der Zentrifuge stattfindet, findet während des Zentrifugiervorganges ein Fernbetätigung durch einen nicht näher dargestellten Betätigungsstößel eines Betätigungszylinders statt, der im Rückteil 15 der Kassette 5 angeordnet ist und der mit seinem Stößel in Pfeilrichtung 63 (Figur 6) auf die Wippe wirkt.

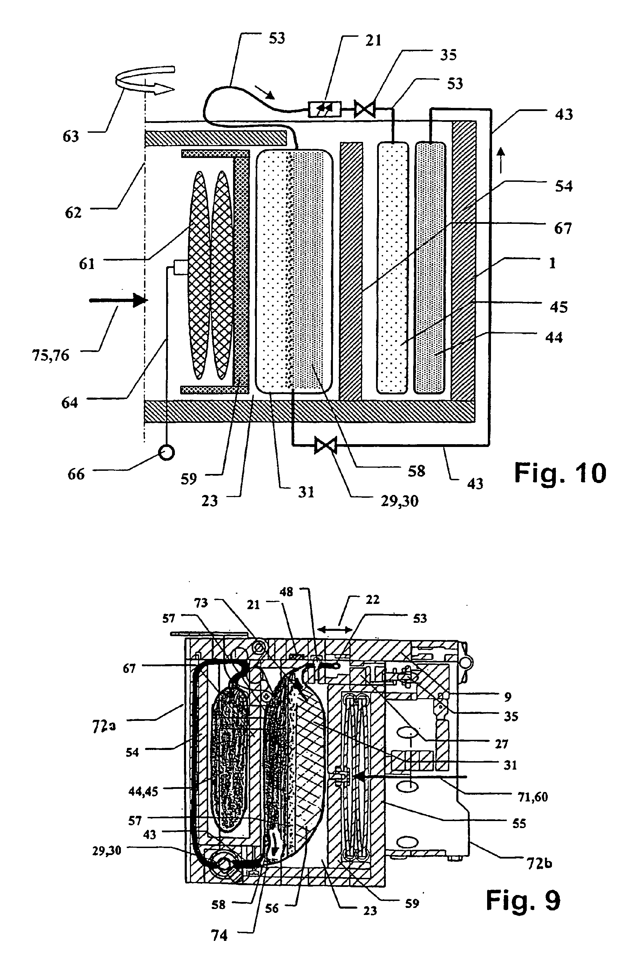

Wichtig ist auch die Darstellung in Figur 9, daß der Blutbeutel 31 nun in seine Kammer 23 so eingelegt ist und seine kopfseitigen und fußseitigen Schlauchabführungen so abgebogen sind, daß er in der Seitenansicht nach Figur 9 eine etwa S-förmige Form einnimmt.

Der obere, zur Gewinnung des Plasmas dienende Schlauch 53 ist mit seinem Schlauchanschluß radial einwärts verformt, so daß er außerhalb der Mittenachse des Vollblutbeutels 31 gelangt, während andererseits in Gegenüberstellung am Fußende der dort abgehende Schlauch 43 wiederum radial auswärts gerichtet ist und auch außerhalb der Längsmittenachse des Vollblutbeutels 31 angeordnet ist.

Diese beiderseits außermittige Anordnung der Schlauchanschlüsse 43, 53 wird nur durch die entsprechende Einlage und Befestigung des Vollblutbeutels 31 in der Kammer 23 erreicht.

Es bedarf hierzu keinerlei Veränderungen am Vollblutbeutel 31 selbst, so daß handelsübliche Vollblutbeutel verwendet werden können.

Damit besteht der Vorteil, daß sich während des Zentrifugiervorganges die in Figur 9 angedeuteten Komponenten bilden.

Radial einwärts bildet sich im Vollblutbeutel 31 das Plasma 56 mit einer relativ scharfen zu dem im Mittenbereich sich ausbildenden Buffycoat 57, während radial auswärts eine stehende und relativ unveränderte Komponente bestehend aus Erythrozyten 58 sich bildet.

Wichtig ist nun, daß die beiden innen- und außenliegenden Komponenten 56, 58 nun störungsfrei in den Pfeilrichtungen 73, 74 nach außen bei entsprechender zeitgesteuerter Öffnung der zugeordneten Klemmen abgeleitet werden können, ohne daß das Buffycoat 57 selbst hierbei irgendwie beeinflußt und mitgenommen wird.

Es verbleibt also im Vollblutbeutel 31 und muß keine anderen Beutel durchlaufen, wie beim Stand der Technik, und verschmutzt deshalb auch keine anderen Beutel, wie es beim Stand der Technik in Kauf genommen werden muß.

Es sind lediglich die beiden Klemmen in Figur 9 im Kopf- und Fußbereich der Schläuche 43, 53 angedeutet. Diese können auch als Rollenschlauchklemmen ausgebildet sein und können anstatt einer Federbelastung auch andere Schließmechanismen aufweisen.

Wichtig hierbei ist nur, daß sie im Arbeitszustand in Schließstellung gehalten werden und nur bedarfsweise bei Anlegung eines entsprechenden Signals in ihrer Offenstellung für einen bestimmten Zeitraum gebracht werden.

Es ist noch in Figur 9 erkennbar, daß die Halterung des Blutbeutels 31 dergestalt erfolgt, daß außerhalb der Mittenlängsachse die Stifte 48 an der Adapterplatte 27 angeordnet sind, die zur Halterung des Blutbeutels 31 dienen.

Je nach Art des verwendeten Blutbeutels 31 kann deshalb die Adapterplatte 27 ausgetauscht werden, um die entsprechenden Schlauchführungskanäle und die Befestigungen mit den Stiften 48 dem Blutbeutel 31 anzupassen.

Gleiches gilt im übrigen für die untenliegenden Stifte 42 im Bodenbereich 28 und die dort bestehenden Durchführungen, die ebenfalls entsprechend dem verwendeten Blutbeutel 31 angepaßt werden können.

Im übrigen ist es bekannt, daß der Vollblutbeutel 31 einen Schlauchstummel 51 trägt, der ebenfalls in die Adapterplatte 27 im Bereich eines Schlauchkanals 49 eingreift. Der für die Gewinnung des Plasmas dienende Plasmaschlauch 53 ist in einen Schlauchkanal 52 in der Adapterplatte 27 geführt. (Figur 6).

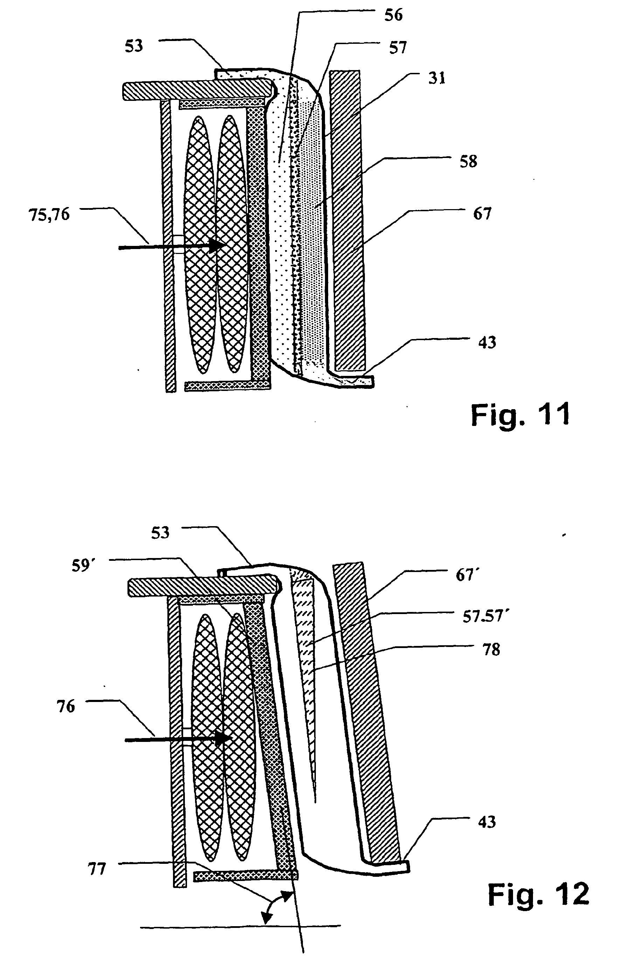

Die Figur 10 zeigt weitere Funktionsteile der Anordnung, wobei die Zuordnung von Kassette 5 und Einsatz 25 nur beispielhaft dargestellt ist.

Es ist nur dargestellt, daß die gesamte Anordnung eine Außenwand 54 aufweist, die radial auswärts an der Innenwand des Rotors 1 anliegt.

Es ist das Mehrkammersystem dargestellt, in dem erkennbar ist, daß in der Kammer 23 der Blutbeutel 31 angeordnet ist.

Radial innen ist ein Preßschieber 59 angeordnet, der bevorzugt im vertikalen Querschnitt U-förmig profiliert ist und der radial nach außen konvex gewölbt ist.

Er wird über einen Schlauch 64 von einem Druckluftanschluß 66 versorgt, wobei dieser Druckluftanschluß 66 über die vorher beschriebenen Steckanschlüsse 3 oder 8 erfolgt.

Beim Aufblasen des gesamten Druckmoduls 61 wird eine Preßkraft in Pfeilrichtung 76 erreicht, die gleichwirkend wie die Zentrifugalkraft 75 ist. (Figur 11).

Auf diese Weise gehört lediglich ein relativ geringes Aufblasen unter geringem Druck dazu, den entsprechenden Preßdruck auf den Beutel 31 aufzubringen.

Es ist im übrigen dargestellt, daß die gesamte Anordnung um die Drehachse 62 beispielsweise in Pfeilrichtung 63 rotiert.

In Figur 11 ist schematisiert die Ableitung der einzelnen Komponenten während des Zentrifugiervorganges dargestellt, wobei erkennbar ist, daß sich eine etwa Z-förmige Lage des Vollblutbeutels 31 ergibt und die sich ergebende Buffycoat-Komponente 57 im Mittenbereich des Blutbeutels 31 aufhält, während die eine Komponente (Plasma) 56 über den Schlauchanschluß 53 abgeleitet wird, und die andere Komponente (Erythrozyten) 58 über den Schlauchanschluß 43 nach unten abgeleitet wird.

Hierbei ist wichtig, daß der Schlauchanschluß 53 nach radial einwärts und der Schlauchanschluß 43 radial auswärts gerichtet ist.

Nur durch diese besondere Z-Formgebung wird erreicht, daß herkömmliche Blutbeutel mit einem oberen und unteren Anschluß in stehender Lage dazu geeignet sind, selektiv die verschiedenen Komponenten 56, 58 nach oben und unten ableiten zu lassen.

In Figur 12 ist ein abgewandeltes Ausführungsbeispiel dargestellt, aus dem erkennbar ist, daß bei einer Schräglage des entsprechenden Preßschiebers 59', der um den Winkel 77 zur Vertikalen geneigt ist, und eine entsprechende Schräglage der dazu gehörenden anliegenden Kammerwand 67' eine keilförmige Ausbildung des dann sich ausbildenden Buffycoats 57 sich ergibt.

Dies hat den wesentlichen Vorteil, daß beim Verschieben des Preßschiebers 59' in Pfeilrichtung 76 zunächst der untere Bereich des Blutbeutels 31 gepreßt wird, so daß sich das Buffycoat 57' weiter oben und in einer sich nach oben verbreitenden Keilform ergibt.

Sobald sich beim Plasmaabfluß der Zustand des Buffycoats 57' oben verbreitert, wie in Figur 12 dargestellt, heißt dies, daß das Plasma nun zur Neige geht, und dies ist ein Zeichen für den oben im Deckelbereich angeordneten Lichtsensor 21, die zugeordnete Klemme 35 zu schließen.

Dieser Zustand ist durch eine entsprechende Mikroprozessorsteuerung zu programmieren und hängt davon ab, auf welche Empfindlichkeit und auf welche Farbveränderung der Lichtsensor eingestellt wird.

Im übrigen kann der Sensor auch noch in radialer Richtung verstellt werden, so wie diese in Pfeilrichtung 22 in Figur 9 angegeben ist.

Der Sensor wird etwa an der Komponentengrenze des Buffycoats 57 zum Plasma 56 positioniert, um eine Verschiebung dieser Grenze in Richtung auf den Plasmaschlauch 53 sofort zu erkennen und die entsprechende Klemme 35 zu schließen.

Im übrigen wird damit sichergestellt, daß ein Restplasmavorrat im Blutbeutel 31 vorgehalten wird, um mit Sicherheit eine Verschmutzung des Plasmabeutels 45 durch das Buffycoat 57 zu vermeiden. Das Restplasma dient auch zur Ernährung der Thrombozyten.

In Figur 9 ist im übrigen auch noch dargestellt, daß der Einsatz 25 noch eine Innenwand 55 aufweist, welche die Kammerwand begrenzt und die jenseits des Preßschiebers 59 angeordnet ist.

Wichtig ist auch noch bei Figur 9 und 10, daß sowohl die Gewichtslast 71 des Preßschiebers 59 als auch die Zentrifugalkraft 75 in Pfeilrichtung 60 radial auswärts gerichtet wirkt, um so eine sehr starke Kraft bei geringem Kraftantrieb des Preßschiebers 59 zu ermöglichen.

Auch ist in Figur 9 noch die radial außen angeordnete Außenwand 72a und die radial innen angeordnete Innenwand 72b der Kassette 5 dargestellt.

Die Keilform des Buffycoats 57' ist mit dem Bezugszeichen 78 in Figur 12 angegeben.

Insgesamt ergibt sich also mit der gegebenen technischen Ausführung der Vorteil, daß nun eine saubere, betriebssichere und von Fremdeingriffen völlig unabhängige Gewinnung von Plasma und Erythrozyten ermöglicht wird, wobei mit handelsüblichen Blutbeutein gearbeitet wird. Die Gewinnung erfolgt vollautomatisch und es bedarf keines Handeingriffes.

Bei der vorliegenden Erfindung ist wesentlich, daß der Blutbeutel in stehender Form so in die Aufnahmekammer eingesetzt wird, daß seine kopfseitigen und fußseitigen Anschlußbereiche außerhalb der Mittenlängsachse (bezogen auf einen geradehängenden, unverformten Blutbeutel) zu liegen kommen. Nur dadurch wird gewährleistet, daß diese Anschlußbereiche sich nicht mehr im Bereich zu der sich im Mittenbereich ausbildenden Buffycoat-Zone befinden.

Hierbei ist es gleichgültig, ob sich der zur Gewinnung des Plasmas dienende Abflußbereich (im Ausführungsbeispiel der Kopfbereich des Blutbeutels) oben oder unten befindet. Es kann der Blutbeutel also auch so eingebaut werden, daß sich der zur Gewinnung des Plasmas befindliche Anschlußschlauch unten befindet und umgekehrt der zur Gewinnung der Erythrozyten dienende Anschlußschlauch sich oben befindet.

Ferner ist nur wesentlich, daß sich die beiden einander gegenüberliegenden Anschlussbereiche diametral gegenüberliegen, d.h., der eine Anschlußbereich weist in entgegengesetzte Richtung zu dem anderen Anschlußbereich. Es ist hierbei gleichgültig, ob beispielsweise der obere Anschlußbereich zur Gewinnung des Plasmas auf einem Radiusstrahl in Richtung zur Drehachse der Zentrifuge zeigt, während der andere diametral gegenüberliegende Anschlußbereich auf einem Radiusstrahl radial auswärts gerichtet von der Nabe des Rotors zeigt.

Es ist selbstverständlich möglich, daß diese Anschlußbereiche nicht genau fluchtend auf einem Radiusstrahl liegen; sie können auch einen Winkel zum Radiusstrahl bilden, vorausgesetzt, sie sind diametral gegenüberliegend und haben jeweils nur Fließverbindung zu der auf der jeweiligen Seite ausbildenden Komponente des Vollblutes.

Statt der vorher beschriebenen Z-Form des Blutbeutels ist also auch eine S-Form möglich und lösungskonform.

Auch die bogenförmige Abführung der Anschlußschläuche 43, 53 vom Blutbeutel 31 ist gestattet.

Auch ist es nicht lösungsnotwendig, daß die Satellitenbeutel 44, 45 auf der gleichen Umfangsebene des Rotors 1 liegen. Sie können durchaus auch auf unterschiedlichen Umfangsebenen angeordnet sein. Gleiches gilt auch für die Anordnung der Kammer für die Aufnahme des Vollblutbeutels. Dieser ist im Ausführungsbeispiel radial einwärts im Vergleich zu den auf gleicher Umfangsebene liegenden radial auswärts gerichteten und dort angeordneten Satellitenbeuteln 44, 45 angeordnet.

Selbstverständlich ist es auch möglich, diesen Vollblutbeutel 31 auf einer radial auswärtsliegenden Umfangsebene im Vergleich zu den mehr radial einwärts angeordneten Satellitenbeuteln 44, 45 anzuordnen.

### Zeichnungslegende

- 1: Rotor
- 2: Nabe
- 3: Steckanschluß
- 4: Innenraum
- 5: Kassette
- 6: Arretierstift
- 7: Bohrung
- 8: Steckanschluß
- 9: Deckel
- 10: Verschlußklappe
- 11: Schwenkachse (Riegel)
- 12: Riegel
- 13: Bolzen
- 14: Schwenkscharnier (Deckel)
- 15: Rückteil
- 16: Schrauben
- 17: Rückwand
- 18: Einstellrad
- 19: Welle
- 20: Führungshülse
- 21: Sensor
- 22: Pfeilrichtung
- 23: Kammer (Blutbeutel)
- 24: Kammer (Plasma)
- 25: Einsatz
- 26: Kammer (Erythrozyt)
- 27: Adapterplatte
- 28: Bodenseite
- 29: Schlauchführungskanal
- 30: Handbetätigung
- 31: Vollblutbeutel
- 32: Pfeilrichtung
- 33: Boden
- 34: Boden
- 35: Handbetätigung (Klemme)
- 36: Wippe
- 37: Schwenkachse
- 38: Klemme
- 39: Führung
- 40: Klemme
- 41: Führung
- 42: Stifte
- 43: Schlauch (Blutbeutel 31)
- 44: Erythrozyten-Beutel
- 45: Plasmabeutel
- 45a: Schlauchführung
- 46: Langloch
- 47: Führungsschraube
- 48: Stift
- 49: Schlauchkanal
- 50: Schlauchkanal
- 51: Schlauchstummel
- 52: Schlauchkanal
- 53: Plasmaschlauch
- 54: Außenwand
- 55: Innenwand
- 56: Plasma
- 57: Buffycoat 57'
- 58: Erythrozyten
- 59: Preßschieber
- 60: Pfeilrichtung
- 61: Druckmodul
- 62: Drehachse
- 63: Pfeilrichtung
- 64: Schlauch
- 66: Druckluftanschluß
- 67: Trennwand 67'
- 68: Anschlagstift
- 69: Betätigungskraft
- 70: Feder
- 71: Gewichtslast
- 72a: Außenwand
- 72b: Innenwand
- 73: Pfeilrichtung
- 74: Pfeilrichtung
- 75: Zentrifugalkraft
- 76: Preßkraft
- 77: Winkel
- 78: Keilform

## Patentansprüche

1. Zentrifuge mit Blutbeutelsystem (31, 44, 45) mit oberem und unterem Abgang zur Trennung von Blutkomponenten, welche Zentrifuge einen um eine Nabe (2) rotierend antreibbaren Rotor (1) beinhaltet, und in diesem Blutbeutelsystem (31, 44, 45) mindestens ein herkömmlicher Vollblutbeutel (31) mit oberem (53) und unteren Abgang (43) stehend angeordnet ist, **dadurch gekennzeichnet, daß** der Vollblutbeutel (31) derart am Rotor (1) angeordnet ist, daß der eine Abgang (43; 53) des Vollblutbeutels (31) in der einen radialen Richtung geknickt ist und der gegenüberliegende Abgang (53; 43) des Vollblutbeutels (31) in der anderen radialen Richtung geknickt ist, so daß insgesamt der Vollblutbeutel (31) in seiner Funktionslage mit seinen Abgängen (43, 53) eine etwa Z- oder S-Form einnimmt.

2. Zentrifuge nach Anspruch 1, **dadurch gekennzeichnet, daß** der obere Abgang (53) des Vollblutbeutels (31) radial nach innen hin zu liegen kommt und der untere Abgang (43) des Vollblutbeutels (31) radial nach außen hin zu liegen kommt.

3. Zentrifuge nach einem der Ansprüche 1 bis 2, **dadurch gekennzeichnet, daß** der obere Abgang (53) des Vollblutbeutels (31) für die Ableitung des Plasmas (56) vorgesehen ist und der untere Abgang (43) des Vollblutbeutels (31) für die Ableitung der Erythrozyten (58).

4. Zentrifuge nach einem der Ansprüche 1 bis 3, **dadurch gekennzeichnet, daß** die Abgänge (43, 53) derart abgeknick sind, daß das Buffycoat (57) während des Ablassens der anderen Blutkomponenten (56, 58) im wesentlichen im Vollblutbeutel (31) selbst verbleibt.

5. Zentrifuge nach einem der Ansprüche 1 bis 4, **dadurch gekennzeichnet, daß** der Vollblutbeutel (31) in einem Einsatz (25) gehalten wird.

6. Zentrifuge nach Anspruch 5, **dadurch gekennzeichnet, daß** der Einsatz (25) in einer Kassette (5) gehalten wird, welche an dem Rotor (1) befestigt ist.

7. Zentrifuge nach Anspruch 6, **dadurch gekennzeichnet, daß** mehrere Kassetten (5) an/in der Zentrifuge vorgesehen sind.

8. Zentrifuge nach Anspruch 7, **dadurch gekennzeichnet, daß** die Kassetten (5) etwa sektorförmig ausgebildet sind und gleichmäßig am Umfang verteilt am Rotor (1) angeordnet sind.

9. Zentrifuge nach einem der Ansprüche 5 bis 8, **dadurch gekennzeichnet, daß** die Kassette (5) lösbar an der Zentrifuge und der Einsatz (25) lösbar an der Kassette (5) angeordnet sind.

10. Zentrifuge nach einem der Ansprüche 5 bis 9, **dadurch gekennzeichnet, daß** die Halterung der Kassette (5) durch mindestens einen Arretierstift (6) erfolgt, welcher radial außen an der Zentrifuge sich befindet.

11. Zentrifuge nach einem der Ansprüche 1 bis 10, **dadurch gekennzeichnet, daß** die Zentrifuge einen Innenraum (4) aufweist, in welcher die mindestens eine Kassette (5) angeordnet ist.

12. Zentrifuge nach einem der Ansprüche 1 bis 11, **dadurch gekennzeichnet, daß** die Kassette (5) einen abnehmbaren bzw. verschwenkbaren, transluzenten oder transparenten Deckel (9) aufweist.

13. Zentrifuge nach einem der Ansprüche 1 bis 12, **dadurch gekennzeichnet, daß** weiterhin mindestens ein Erythrozyten-Beutel (44) und mindestens ein Plasmabeutel (45) an/in der Zentrifuge vorgesehen sind.

14. Zentrifuge nach Anspruch 13, **dadurch gekennzeichnet, daß** auch die anderen beiden Beutel (44, 45) in dem Einsatz (25) gehalten werden können.

15. Zentrifuge nach Anspruch 14, **dadurch gekennzeichnet, daß** jeder der Beutel (31, 44, 45) in eine separaten Kammer (23, 24, 26) angeordnet ist.

16. Zentrifuge nach einem der Ansprüche 5 bis 15, **dadurch gekennzeichnet, daß** der Einsatz (25) mit entsprechend gesteuerten Klemmen (38, 40) versehen ist, welche in Abhängigkeit des Zentrifugierstadiums geöffnet oder geschlossen werden können und somit die Überleitung der im Vollblutbeutel (31) hergestellten Komponenten (56-58) in die einzelnen Satelliten-Beutel (31, 44, 45) durch klemmenlöffnen der Abgänge (43, 53) ermöglicht wird.

17. Zentrifuge nach Anspruch 16, **dadurch gekennzeichnet, daß** die Klemmen (38, 40) federbelastet die Abgänge (43, 53) geschlossen halten und im Betrieb der Zentrifuge programmgesteuert geöffnet werden können.

18. Zentrifuge nach einem der Ansprüche 16 oder 17, **dadurch gekennzeichnet, daß** Klemmen (38,40) in Abhängigkeit eines Sensors (21) betätigt werden.

19. Zentrifuge nach Anspruch 18, **dadurch gekennzeichnet, daß** der Sensor (21) die Menge an Buffycoat (57) ermittelt.

20. Zentrifuge nach Anspruch 19, **dadurch gekennzeichnet, daß** der Sensor (21) die Menge an Buffycoat (57) anhand der Breite und/oder Färbung der Buffycoat-Schicht im oberen Randbereich des Vollblutbeutels (31) bestimmt.

21. Zentrifuge nach einem der Ansprüche 16 bis 20, **dadurch gekennzeichnet, daß** der Sensor (21) etwa radial verfahrbar ausgebildet ist.

22. Zentrifuge nach einem der Ansprüche 16 bis 21, **dadurch gekennzeichnet, daß** die Ansteuerungselemente für das Ansteuern der Klemmen (38, 40) in der Kassette (5) angeordnet sind.

23. Zentrifuge nach einem der Ansprüche 1 bis 22, **dadurch gekennzeichnet, daß** die Energie- und/oder Medienanschlüsse über die Nabe (2) erfolgt.

24. Zentrifuge nach einem der Ansprüche 1 bis 23, **dadurch gekennzeichnet, daß** eine Schweißvorrichtung vorgesehen ist, welche die Beutel (31, 44, 45) hermetisch dichtend zuschweißen kann.

25. Zentrifuge nach einem der Ansprüche 1 bis 24, **dadurch gekennzeichnet, daß** ein radial innen liegendes Druckmodul (61) vorgesehen ist, welches radial nach außen hin auf den Vollblutbeutel (31) arbeiten und diesen mit Druck beaufschlagen kann.

26. Zentrifuge nach Anspruch 25, **dadurch gekennzeichnet, daß** das Druckmodul (61) einen ein mit Preßluft aufblasbares Kissen beinhaltet, welcher einen Preßschieber (59) bewegen kann, der am Vollblutbeutel (31) angeordnet ist.

## Claims

1. Centrifuge with blood bag system (31, 44, 45) with an upper and lower outlet for the separation of blood components, which centrifuge contains a rotor (1) which can be driven so as to rotate about a hub (2), and at least one conventional whole blood bag (31) with an upper (53) and lower outlet (43) is arranged vertically in this blood bag system (31, 44, 45), **characterised in that** the whole blood bag (31) is arranged on the rotor (1) in such a way that one outlet (43; 53) of the whole blood bag (31) is bent in one radial direction and the opposing outlet (53; 43) of the whole blood bag (31) is bent in the other radial direction, so overall the whole blood bag (31) in its functional position, with its outlets (43, 53), adopts an approximately Z- or S-shape.

2. Centrifuge according to claim 1, **characterised in that** the upper outlet (53) of the whole blood bag (31) comes to rest radially inwardly and the lower outlet (43) of the whole blood bag (31) comes to rest radially outwardly.

3. Centrifuge according to any one of claims 1 to 3, **characterised in that** the upper outlet (53) of the whole blood bag (31) is provided for the withdrawal of plasma (56) and the lower outlet (43) of the whole blood bag (31) is provided for the withdrawal of erythrocytes (58).

4. Centrifuge according to any one of claims 1 to 4, **characterised in that** the outlets (43, 53) are bent in such a way that the buffy coat (57) remains substantially in the whole blood bag (31) itself during the discharge of the other blood components (56, 58).

5. Centrifuge according to any one of claims 1 to 4, **characterised in that** the whole blood bag (31) is held in an insert (25).

6. Centrifuge according to claim 6, **characterised in that** the insert (25) is held in a cartridge (5), which is fastened to the rotor (1).

7. Centrifuge according to claim 7, **characterised in that** a plurality of cartridges (5) are provided on/in the centrifuge.

8. Centrifuge according to claim 8, **characterised in that** the cartridges (5) are configured approximately in a sector shape and are arranged on the rotor (1), uniformly distributed on the periphery.

9. Centrifuge according to any one of claims 6 to 9, **characterised in that** the cartridge (5) is arranged detachably on the centrifuge and the insert (25) is arranged detachably on the cartridge (5).

10. Centrifuge according to any one of claims 6 to 10, **characterised in that** the cartridge (5) is held by at least one locking pin (6), which is located radially outwardly on the centrifuge.

11. Centrifuge according to any one of claims 1 to 11, **characterised in that** the centrifuge has an interior (4), in which the at least one cartridge (5) is arranged.

12. Centrifuge according to any one of claims 1 to 12, **characterised in that** the cartridge (5) has a removable or pivotable, translucent or transparent lid (9).

13. Centrifuge according to any one of claims 1 to 13, **characterised in that**, furthermore, at least one erythrocyte bag (44) and at least one plasma bag (45) are arranged on/in the centrifuge.

14. Centrifuge according to claim 14, **characterised in that** the other two bags (44, 45) can also be held in the insert (25).

15. Centrifuge according to claim 15, **characterised in that** each of the bags (31, 44, 45) is arranged in a separate chamber (23, 24, 26).

16. Centrifuge according to any one of claims 6 to 16, **characterised in that** the insert (25) is provided with appropriately controlled clamps (38, 40), which can be opened or closed as a function of the centrifuging stage and therefore the transfer of the components (56 to 58) produced in the whole blood bag (31) into the individual satellite bags (31, 44, 45) is made possible by clamping/opening the outlets (43, 53).

17. Centrifuge according to claim 17, **characterised in that** the clamps (38, 40) keep the outlets (43, 53) closed in a spring-loaded manner and can be opened in a programme-controlled manner during operation of the centrifuge.

18. Centrifuge according to either of claims 17 or 18, **characterised in that** clamps (38, 40) are actuated as a function of a sensor (21).

19. Centrifuge according to claim 19, **characterised in that** the sensor (21) determines the quantity of buffy coat (57).

20. Centrifuge according to claim 20, **characterised in that** the sensor (21) determines the quantity of buffy coat (57) with the aid of the width and/or colouring of the buffy coat layer in the upper edge region of the whole blood bag (31).

21. Centrifuge according to any one of claims 17 to 21, **characterised in that** the sensor (21) is configured so as to be approximately radially displaceable.

22. Centrifuge according to any one of claims 17 to 22, **characterised in that** the activation elements for activation of the clamps (38, 40) are arranged in the cartridge (5).

23. Centrifuge according to any one of claims 1 to 23, **characterised in that** the power and/or media connections take place via the hub (2).

24. Centrifuge according to any one of claims 1 to 24, **characterised in that** a welding device is provided, which can seal the bags (31, 44, 45) closed in a hermetically sealing manner.

25. Centrifuge according to any one of claims 1 to 25, **characterised in that** a radially inwardly located pressure module (61) is provided, which can operate radially outwardly on the whole blood bag (31) and can apply pressure thereto.

26. Centrifuge according to claim 26, **characterised in that** the pressure module (61) contains a cushion which can be inflated with compressed air and can move a pressure slide (59), which is arranged on the whole blood bag (31).

## Revendications

1. Centrifugeuse avec un système de poche de sang (31, 44, 45) présentant une sortie supérieure et une sortie inférieure pour la séparation des composants du sang, ladite centrifugeuse contenant un rotor (1) qui est apte à être entraîné en rotation autour d'un moyeu (2), et au moins une poche de sang entier (31) classique avec des sorties supérieure (53) et inférieure (43) étant disposée à la verticale dans ce système de poche de sang (31, 44, 45), **caractérisée en ce que** la poche de sang entier (31) est disposée sur le rotor (1) de telle sorte qu'une sortie (43 ; 53) de la poche de sang entier (31) soit coudée dans un sens radial tandis que la sortie opposée (53 ; 43) est coudée dans l'autre sens radial, de sorte que dans l'ensemble, la poche de sang entier (31) prend en position fonctionnelle, avec ses sorties (43, 53), une forme à peu près en Z ou en S.

2. Centrifugeuse selon la revendication 1, **caractérisée en ce que** la sortie supérieure (53) de la poche de sang entier (31) vient se placer radialement vers l'intérieur tandis que la sortie inférieure (43) vient se placer radialement vers l'extérieur.

3. Centrifugeuse selon la revendication 1 ou 2, **caractérisée en ce que** la sortie supérieure (53) de la poche de sang entier (31) est prévue pour l'évacuation du plasma (56), et la sortie inférieure (43) pour l'évacuation des érythrocytes (58).

4. Centrifugeuse selon l'une des revendications 1 à 3, **caractérisée en ce que** les sorties (43, 53) sont coudées de telle sorte que la couche leuco-plaquettaire (57) reste quasiment dans la poche de sang entier (31) elle-même pendant l'évacuation des autres composants du sang (56, 58) .

5. Centrifugeuse selon l'une des revendications 1 à 4, **caractérisée en ce que** la poche de sang entier (31) est maintenue dans un élément rapporté (25).

6. Centrifugeuse selon la revendication 5, **caractérisée en ce que** l'élément rapporté (25) est maintenu dans une cassette (5) qui est fixée au rotor (1).

7. Centrifugeuse selon la revendication 6, **caractérisée en ce que** plusieurs cassettes (5) sont prévues sur/dans la centrifugeuse.

8. Centrifugeuse selon la revendication 7, **caractérisée en ce que** les cassettes (5) ont à peu près la forme de secteurs et sont répartis régulièrement sur la circonférence du rotor (1).

9. Centrifugeuse selon l'une des revendications 6 à 8, **caractérisée en ce que** la cassette (5) est disposée de manière amovible sur la centrifugeuse et l'élément rapporté (25) est disposé de manière amovible sur la cassette (5).

10. Centrifugeuse selon l'une des revendications 6 à 9, **caractérisée en ce que** la fixation de la cassette (5) se fait à l'aide d'au moins une tige de blocage (6) qui est située radialement sur l'extérieur de la centrifugeuse.

11. Centrifugeuse selon l'une des revendications 1 à 10, **caractérisée en ce qu'**elle présente un espace intérieur (4) dans lequel sont disposées la ou les cassettes (5).

12. Centrifugeuse selon l'une des revendications 1 à 8, **caractérisée en ce que** la cassette (5) comporte un couvercle (9) amovible ou pivotant, translucide ou transparent.

13. Centrifugeuse selon l'une des revendications 1 à 12, **caractérisée en ce qu'**il est également prévu au moins une poche d'érythrocytes (44) et au moins une poche de plasma (45) sur/dans la centrifugeuse.

14. Centrifugeuse selon la revendication 13, **caractérisée en ce que** les deux autres poches (44, 45) peuvent être maintenues elles aussi dans l'élément rapporté (25) .

15. Centrifugeuse selon la revendication 14, **caractérisée en ce que** chacune des poches (31, 44, 45) est disposée dans une chambre distincte (23, 24, 26).

16. Centrifugeuse selon l'une des revendications 1 à 15, **caractérisée en ce que** l'élément rapporté (25) est pourvu de pinces (38, 40) commandées d'une manière appropriée, qui peuvent s'ouvrir ou se fermer en fonction du stade de la centrifugation, et le transfert des composants (56-58) fabriqués dans la poche de sang entier (31) dans les poches satellites individuelles (31, 44, 45) est ainsi possible grâce au serrage/à l'ouverture des sorties (43, 53).

17. Centrifugeuse selon la revendication 16, **caractérisée en ce que** les pinces (38, 40), contraintes par ressort, maintiennent les sorties (43, 53) fermées et peuvent s'ouvrir en étant commandées par un programme, pendant le fonctionnement de la centrifugeuse.

18. Centrifugeuse selon la revendication 16 ou 17, **caractérisée en ce que** les pinces (38, 40) sont actionnées en fonction d'un capteur (21).

19. Centrifugeuse selon la revendication 18, **caractérisée en ce que** le capteur (21) détermine la quantité de couche leuco-plaquettaire (57).

20. Centrifugeuse selon la revendication 1 à 19, **caractérisée en ce que** le capteur (21) définit la quantité de couche leuco-plaquettaire (57) à l'aide de la largeur et/ou de la coloration de celle-ci dans la zone du bord supérieur de la poche de sang entier (31).

21. Centrifugeuse selon l'une des revendications 16 à 20, **caractérisée en ce que** le capteur (21) est conçu pour être mobile radialement.

22. Centrifugeuse selon l'une des revendications 16 à 21, **caractérisée en ce que** les éléments de commande pour la commande des pinces (38, 40) sont disposés dans la cassette (5).

23. Centrifugeuse selon l'une des revendications 1 à 22, **caractérisée en ce que** les raccordements d'énergie et/ou d'agents se font par l'intermédiaire du moyeu (2).

24. Centrifugeuse selon l'une des revendications 1 à 23, **caractérisée en ce qu'**il est prévu un dispositif de soudage qui peut souder les poches (31, 44, 45) pour les fermer hermétiquement.

25. Centrifugeuse selon l'une des revendications 1 à 24, **caractérisée en ce qu'**il est prévu un module de pression (61) placé radialement à l'intérieur, qui peut agir sur la poche de sang entier (31) radialement vers l'extérieur et soumettre celle-ci à une pression.

26. Centrifugeuse selon la revendication 25, **caractérisée en ce que** le module de pression (61) contient un coussin gonflable avec de l'air comprimé, qui peut déplacer un coulisseau presseur (59) disposé sur la poche de sang entier (31).
